# EUROPEAN PATENT APPLICATION

(11) **EP 4 678 753 A1**
(43) Date of publication of application: **14.01.2026**
(21) Application number: 24766433.7
(22) Date of filing: 05.03.2024
(51) Int. Cl.: C12N 15/85, C12N 15/113, A61K 39/00, A61P 35/00

(54) **PREPARATION METHOD FOR ANTIGEN EPITOPE**

(30) Priority: 08.03.2023 CN 202310222094
(71) Applicant: GUANGZHOU MEDICAL UNIVERSITY, Guangzhou, Guangdong 511436 (CN)
(72) Inventor: WANG, Xiaoling, Guangzhou, Guangdong 511436 (CN); CHEN, Lin, Guangzhou, Guangdong 511436 (CN)
(74) Representative: IP Sextant s.r.l.
(86) International application number: PCT/CN2024/080134
(87) International publication number: WO 2024/183718

(57) **Abstract**

Disclosed is a preparation method for an antigenic epitope. The preparation method comprises the following step: using RNA editing to perform site-directed mutagenesis on a tumor antigen to obtain a neoantigen epitope. The generated neoantigen has the sharing property, avoiding the inaccuracy of identifying a tumor personalized neoantigen, and reducing development costs. An RNA editing system that is safer and more controllable than the DNA editing is used, the editing targets mRNA rather than genomic DNA, and an editing result is reversible and controllable.

## Description

### TECHNICAL FIELD

The present invention belongs to the field of biotechnology, and in particular, relates to a preparation method for an antigen epitope.

### BACKGROUND

Tumor immunotherapy activates the self immune system of cancer patients to kill tumors, which was ranked as the top ten scientific breakthroughs by the journal Science in 2013, and had an optimistic outlook. The main types of tumor immunotherapy include technologies such as immune checkpoint inhibitors, CAR-T cell therapy technology, TCR-T cell therapy technology, and therapeutic neoantigen tumor vaccines. Among them, the immune checkpoint inhibitors Opdivo and Keytruda have ranked as the 2^{nd} and 4^{th}, in terms of sales of drugs for cancer treatment in 2022. The core of immunotherapy is to enhance T-cell response to tumor antigens. Tumor antigens can be classified into tumor-associated antigens (TAA) and tumor-specific antigens (TSA). TSA includes viral antigens and neoantigens generated by mutation of non-synonymous somatic cells, while TAA includes tissue-specific antigens and developmental-specific antigens. TAA are aberrantly or overexpressed in tumors but are still expressed in normal tissues, thus, targeting TAA may result in toxic side effects; additionally, the immune system is in a state of central tolerance to self-antigens, and the immune response induced by TAA is relatively weak. Therefore, tumor neoantigens derived from tumor cell mutations are ideal targets for tumor immunotherapy. Through presentation by the major histocompatibility complex (MHC), neoantigens can induce the activation of helper T cells and killer T cells to generate specific immune response against tumor cells. The immune response to neoantigens has been found to be one of the key factors determining the efficacy of immunotherapy. Immune checkpoint inhibitors are more effective for tumor types with microsatellite instability and high mutation burden (e.g., melanoma, small-cell lung cancer, bladder cancer, etc.), and are often more effective for patients with a high number of mutations, even within the same tumor type.

As a type of TSA, neoantigens exhibit characteristics of high tumor specificity and strong immunogenicity, making them ideal targets for cancer therapy. TCR-T therapy targeting the neoantigen KrasG12 has demonstrated promising clinical outcomes. This mutation is a tumor-driven mutation expressed in 90% of pancreatic cancers, 50% of colon cancers and 25% of lung cancers. However, similar neoantigenic epitopes that can be shared are rare, making it difficult to develop a systematic treatment strategy. Therefore, neoantigen vaccines, which are at the forefront of tumor therapeutic research, currently adopt a personalized treatment strategy. The development of next-generation sequencing technology and algorithms for predicting MHC-binding epitopes in the last decade has paved the way for the development of neoantigen vaccines. A widely adopted approach involves using exon and RNA sequencing, software prediction and validation of neoantigenic epitopes efficiently presented by HLA, followed by using minigenes, RNA, or direct synthesis of short peptides, to tailor personalized neoantigen vaccines for patients. In 2015, results of the clinical trial of tumor neoantigen vaccine were published for the first time in journal Science, confirming that the neoantigen DC vaccine can effectively activate antigen-specific T cells in melanoma. Several subsequent similar studies have shown clinical efficacy on melanoma, glioma, and other cancer patients, with efficacy significantly better than earlier tumor vaccines targeting TAA. In addition, the neoantigen vaccine in combination with PD-1 inhibitors is able to treat advanced or metastatic melanoma, non-small cell lung cancer, and bladder cancer, achieving prolonged and consistently improved progression-free survival.

However, therapies based on neoantigens also have insurmountable drawbacks. Firstly, neoantigens have personalized characteristics, making them difficult to be shared. The types of neoantigens vary from patient to patient, and the production costs for detecting, designing and synthesizing neoantigen vaccines for each patient far exceed those of other types of immunotherapy. Secondly, the accuracy of neoantigen prediction is not high. Current neoantigen prediction technology can only predict the affinity of mutated antigens for MHC-I, rather than predicting actual level of induced T-cell response level. In addition to short peptide-MHC affinity, the quality of neoantigens also includes degree of exogeneity (i.e., measuring the degree of heterogeneity of the neoantigens compared to wild-type proteins), clonal distribution, mutation status, and the affinity of TCR-MHC complexes. In two clinical trials of tumor neoantigens, in which patients received 20 tumor neoantigen peptides and 10 RNA drugs of tumor neoantigen, respectively, only 25% and 16% of the tumor neoantigens elicited specific CD8+ T cell response, respectively. Thirdly, most types of cancer have low mutation burden and lack of expression of new antigens, making vaccine design difficult.

On the other hand, the immunogenicity of neoantigens is also regulated by "immunoediting", in which tumors can escape from immune surveillance by downregulating highly immunogenic neoantigens. Dr. Schreiber proposed the theory of immunoediting in 2002, which posits that the relationship between the immune system and tumors exists in three states: early immune clearance, mid-stage immune equilibrium and late immune escape. This theory has been gradually validated in recent years. Rooney et al. found that the number of mutated short peptides capable of binding to MHC class I molecules in the rectal cancer sample was far lower than the theoretical value, suggesting that the immune system is under pressure to screen for neoantigens. The neoantigen profile in tumors is subjected to immune surveillance to present a dynamic process. Luksza et al. conducted a long-term tracking analysis of neoantigen-specific T cell response in pancreatic ductal adenocarcinoma, and published their findings in journal Nature in 2022, which shows that tumors may downregulate gene expression or delete immunogenic neoantigens at the DNA level.

In summary, the potential for widespread application of neoantigens is limited by the drawbacks of neoantigen prediction technology, low percentage of neoantigens expressed in some patients, high cost of personalized production and the difficulty in establishing quality standards. In addition, the immunogenicity of naturally-occurring neoantigens in tumors is low under the regulation of "immunoediting", and there is an urgent need to develop more broadly applicable and effective neoantigen-related technologies. Currently, some attempts have been made with in situ tumor vaccines, such as Flt3L, GM-CSF, and TLR immune agonists that promote dendritic cell maturation, whose primary mechanism is to promote neoantigen presentation and T-cell activation. Some research groups have innovatively used approaches of oncolytic viruses, bacteria, and chemotherapy to increase neoantigens, but significant challenges remain in terms of tumor targeting, controllability, and specificity.

### SUMMARY

The present invention is intended to solve at least one of the above-mentioned technical problems in the prior art. To this end, the present invention provides a preparation method for an antigenic epitope.

The present invention also provides an antigenic epitope obtained by the preparation method described above.

According to an aspect of the present invention, a preparation method for an antigenic epitope is provided, the preparation method comprises the following step: using RNA editing technology to perform site-directed mutation on a tumor antigen to obtain a neoantigenic epitope.

In some embodiments of the present invention, the site-directed mutation is achieved by mutating A to I and/or C to U in a nucleic acid sequence of RNA of the tumor antigen.

In some embodiments of the present invention, the tumor antigen comprises a tumor-specific antigen or a tumor-associated antigen.

In some embodiments of the present invention, the tumor-specific antigen comprises at least one of a chemically-induced tumor-specific antigen, a viral-induced tumor-specific antigen, and an antigen of a spontaneous tumor.

In some embodiments of the present invention, the tumor-associated antigen comprises at least one of an embryonic antigen, a differentiation antigen, a cancer testis antigen, and a viral antigen.

In some embodiments of the present invention, the tumor comprises at least one of testicular cancer, rectal cancer, colorectal cancer, small intestine cancer, colorectal cancer, gastric cancer, esophageal cancer, hypopharyngeal cancer, laryngeal cancer, oral cancer, nasal cancer, pancreatic cancer, hepatocellular cancer, lung cancer, breast cancer, ovarian cancer, cervical cancer, endometrial cancer, vaginal cancer, fallopian tube cancer, kidney cancer, melanoma, brain tumor, thyroid cancer, parathyroid cancer, leukemia, lymphoma, myeloma, sarcoma, prostate cancer, bladder cancer, cholangiocarcinoma, and gallbladder cancer.

In some embodiments of the present invention, an epitope of the tumor-associated antigen comprises P1A, AFP, or Spag9-2.

In some embodiments of the present invention, an editing system employed by the RNA editing comprises at least LEAPER, λN-BoxB, or MS2-MCP2.

In some embodiments of the present invention, the editing system comprises a guide RNA construct (guide RNA); the guide RNA has a sequence of not less than 16 bp, is reverse complementary to a sequence of a mRNA target site, and has a homology of not less than 85%.

In some embodiments of the present invention, the construct comprises a plasmid, a viral vector, or an RNA.

In some embodiments of the present invention, the RNA comprises a modified RNA and a cyclic RNA.

In some embodiments of the present invention, a sequence of the guide RNA is shown as SEQ ID NO: 5, SEQ ID NO: 6, SEQ ID NO: 7, SEQ ID NO: 12, SEQ ID NO: 19, SEQ ID NO: 20 or SEQ ID NO: 21.

In some embodiments of the present invention, the method further comprises a step of detecting the antigenic epitope obtained from the preparation, the detecting comprises sequence analysis of the antigenic epitope.

In some embodiments of the present invention, a method for the sequence analysis is sequencing.

In some embodiments of the present invention, an affinity of the neoantigenic epitope for binding to MHC is not less than 1000 nM.

In a second aspect of the present invention, an antigenic epitope prepared by the method described above is provided.

In some embodiments of the present invention, an amino acid sequence of the antigenic epitope is shown as SEQ ID NO: 3, SEQ ID NO: 11, SEQ ID NO: 16, SEQ ID NO: 17 or SEQ ID NO: 18.

In a third aspect of the present invention, use of the antigenic epitope described above is provided, the use refers to an use in preparation of a product for treating and/or preventing tumor.

In some embodiments of the present invention, the use refers to the use in the preparation of tumor immunization product.

In some embodiments of the present invention, the use refers to the use in the preparation of a tumor-associated vaccine.

According to some embodiments of the present invention, at least the following beneficial effects are achieved: the technical scheme of the present invention utilizes RNA editing to perform site-directed mutation on a tumor antigen, thereby obtaining a neoantigenic epitope, which avoids inaccuracy in the prediction of neoantigens and reduces the time and economic cost of the prediction; by selecting tumor antigens that are shared for expression in the tumor cells but not expressed in normal tissues, the artificially introduced neoantigens exhibit both tumor specificity and shared expression; using a safer and more controllable RNA editing system than DNA editing, the editing targets mRNA rather than genomic DNA, and the editing results are reversible and controllable; meanwhile, current research on tumor antigen is restricted by central tolerance to this type of antigen, the present invention introduces a point mutation into this type of antigen, and the mutated antigenic epitope is not restricted by central tolerance; under immunoediting pressure, the current neoantigen therapy may exhibit reduced responsiveness, whereas the technical scheme of the present invention allows for the flexible introduction of neoantigens at any time during tumorigenesis and development, thereby enhancing the responsiveness of immunotherapy.

### BRIEF DESCRIPTION OF DRAWINGS

The present invention is further described below in conjunction with the drawings and embodiments, wherein:
FIG. 1 shows a flowchart illustrating the design and detection of the RNA editing-generated mutant peptide REISN in Example 1 of the present invention;
FIG. 2 shows a diagram of the lentiviral vector pLKO-P1A-P2A-EGFP in Example 2 of the present invention;
FIGs. 3A-3C are respective graphs showing the editing efficiency results of the P1A guide RNA designed for three systems (LEAPER, λN-BoxB, and MS2-MCP2) in Example 2 of the present invention;
FIG. 4 is a graph showing the result of successful RNA editing of the P1A epitope of Example 2 of the present invention in CT26 cells;
FIG. 5 shows a diagram of the Dox-inducible ADAR lentiviral vector in Example 2 of the present invention;
FIG. 6 is a graph showing the validation results of the Dox-inducible RNA of Example 2 of the present invention in editing cell lines;
FIGs. 7A and 7B are respective graphs showing the detection results of intratumorally induced RNA editing efficiency in the normal mouse diet group and the dox-added mouse diet group in Example 2 of the present invention;
FIG. 8 shows a flowchart of immunotherapy using the REISN vaccine in Example 2 of the present invention;
FIG. 9 is a graph showing the result of the impact of the REISN vaccine in Example 2 of the present invention on proliferation of tumor;
FIGs. 10A and 10B are graphs showing the detection results of intratumorally induced RNA editing efficiency in the dox-free group and the REISN group in Example 3 of the present invention;
FIG. 11 is a graph showing the result of the impact of the REISN vaccine in Example 3 of the present invention on proliferation of tumor;
FIGs. 12A-12C are respective graphs showing the detection results of editing efficiency of AFP guide RNA designed for three systems (LEAPER, λN-BoxB, and MS2-MCP2) in Example 4 of the present invention.

### DETAILED DESCRIPTION

The following will provide a clear and complete description for the concept of the present invention and the generated technical effects in conjunction with the examples, so as to fully understand the purpose, features and effects of the present invention. Obviously, the described examples are only a part of the examples of the present invention rather than all of them. Based on the examples of the present invention, other embodiments obtained by those skilled in the art without creative labor all fall within the scope of protection of the present invention.

### Example 1

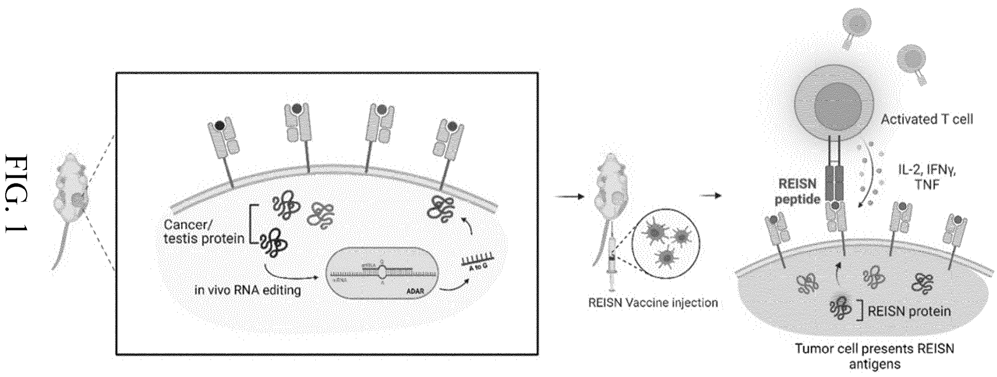

The present example provided a preparation method for an antigenic epitope, the principle of which was shown in FIG. 1, and a specific process was as follows:
(1) for a gene of tumor antigen, NetMHCpan-4.1 software was used to predict a short peptide in the gene with a high binding affinity for a corresponding MHC;
(2) from the adenine nucleotides of the nucleotide sequence of the short peptide screened in (1), a mutant peptide that could change the amino acid coding after mutating to guanine, and whose binding affinity for the MHC was predicted to be not less than 1000 nM by the NetMHCpan-4.1 software was identified, site-directed RNA editing point mutation was performed on the mutant peptide, and a neoantigenic epitope (a mutant peptide) was obtained;
(3) detection was performed on the neoantigenic epitope, specifically comprising:
   1) design and validation of guide RNA: guide RNAs were designed based on an RNA editing system (MS2-MCP2, λN-BoxB or LEAPER editing system), RNA editing efficiency at a corresponding target was validated by in vitro transfection of a mammalian cell;
   2) vaccine was prepared based on the neoantigenic epitope and the anti-tumor effect was validated.

### Example 2

The present example tested the preparation method for the antigenic epitope of Example 1 by using murine cancer testis antigen P1A as a target, comprising the following steps of:

### 1. Design of REISN site

Murine cancer testis antigen target P1A was used as a target, sequence of the antigenic epitope predicted by NetMHCpan-4.1 was shown as SEQ ID NO: 1, and a corresponding RNA sequence was shown as SEQ ID NO: 2. Among the adenine nucleotides in this peptide segment, a short REISN peptide (sequence: LPCLGWLVF (SEQ ID NO: 3)) that could change the amino acid coding after mutating to guanine, and with a binding affinity for the MHC of above 1000 nM was identified. Tumor neoantigen (RNA Editing-Induced Site-Specific Neoantigen, REISN) was generated after inducing RNA editing, with a sequence shown as SEQ ID NO: 3, and a corresponding RNA sequence shown as SEQ ID NO: 4. The neoantigenic epitope predicted by NetMHCpan-4.1 had a binding affinity of 437.34 nM for H-2-Ld, and the specific sequences were as shown in Table 1.

**Table 1**

| Protein name | Peptide | Length | MHC | Aff(nM) | Corresponding RNA sequence |
|---|---|---|---|---|---|
| P1A | LPYLGWLVF ( SEQ ID NO: 1) | 9-mer | H-2-Ld | 56.96 | CUGCCUUAUCUAGGGUGGC UGGUCUUC（SEQ ID NO: 2） |
| P1A-REISN | LPCLGWLVF ( SEQ ID NO: 3) | 9-mer | H-2-Ld | 437.34 | CUGCCUUgUCUAGGGUGGCU GGUCUUC（SEQ ID NO: 4） |

### 2. Design and validation of REISN guide RNA

For the design of guide RNA targeting the P1A site of SEQ ID NO: 2, three systems was referenced and compared:
(1) Guide RNA design based on MS2-MCP2 system: MS2-MCP2 system consisted of two components: exogenous ADAR1 and guide RNA (sequences shown as SEQ ID NO: 5 in Table 2).

Two plasmids of pcDNA3.1(-)-U6-MCP-ADAR1-TAG and pUC57-arP1A-MS2 were constructed. The pcDNA3.1(-)-U6-MCP-ADAR1-TAG plasmid and arP1A-MS2 gene were both synthesized by Nanjing GenScript Biotechnology Co., Ltd.

The pcDNA3.1(-)-U6-MCP-ADAR1-TAG plasmid was constructed by inserting U6-MCP-ADAR1-TAG sequence (sequence shown as SEQ ID NO: 24) between EcoRI and BamHI of the pcDNA3.1 vector.

The pUC57-arP1A-MS2 was constructed by inserting U6-arP1A-MS2 fragment (sequence shown as SEQ ID NO: 25) between restriction enzyme cutting sites of EcoRI and HindIII in the MCS region of the pUC57 vector. Sequencing verified that the sequence was correct and complete.

(2) Guide RNA design based on LEAPER system: LEAPER editing system consisted of guide RNA (sequence shown as SEQ ID NO: 6 in Table 2) containing a structural domain that can recruit endogenous ADAR.

The LEAPER editing system consisted of guide RNA (i.e. ar16, sequence shown as SEQ ID NO: 6). pLKO-p1a-ar16 plasmid was constructed. This plasmid was constructed by cloning U6 promoter and the synthesized ar16 fragment (sequence shown as SEQ ID NO: 6) into between XbaI and SalI of pLKO.1 puro (Addgene #8453) backbone via homologous recombination.

(3) Guide RNA design based on λN-BoxB system: λN-BoxB editing system consisted of exogenous ADAR2 and guide RNA (sequence shown as SEQ ID NO: 7 in Table 2), and was constructed to obtain pUC57-arP1A-BOXB, and pcDNA3.1(-)-U6-arP1A-BOXB-ADAR2 plasmids, both of which were synthesized by Nanjing GenScript Biotechnology Co., Ltd.

Wherein, the pcDNA3.1(-)-U6-arP1A-BOXB-ADAR2 was generated by homologous recombination of pcDNA3.1(-)-myc-HisA, and by inserting λN-ADAR2 (sequence shown as SEQ ID NO: 26) fragment behind the CMV promoter of the backbone, and inserting arP1A-BOXB (sequence shown as SEQ ID NO: 7 in Table 2) behind the U6 promoter. The pUC57-arP1A-BoxB was obtained by cloning the U6 promoter, guide RNA (sequence shown as SEQ ID NO: 7 in Table 2) between restriction enzyme cutting sites of EcoRI and HindIII in the pUC57 backbone. Sequencing verified that the sequence was correct and complete.

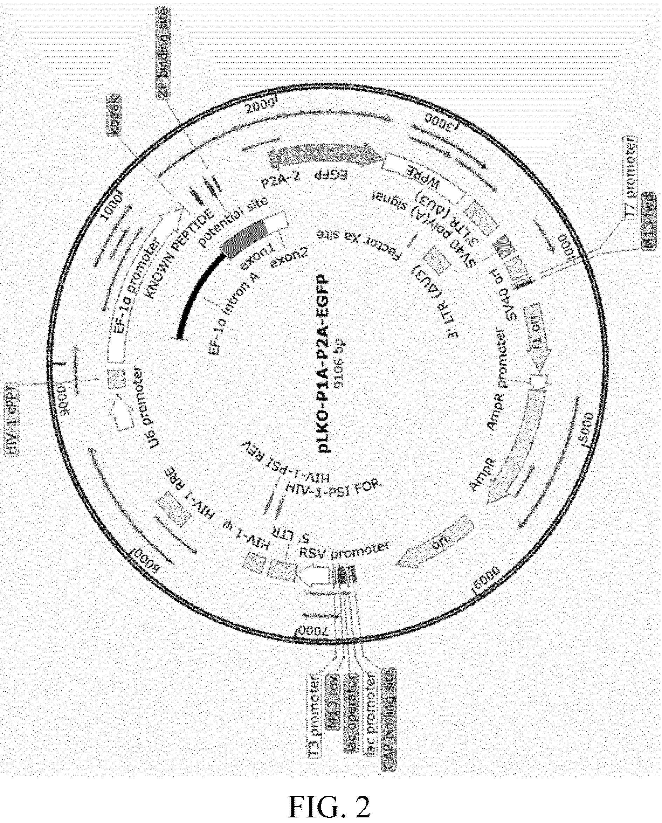

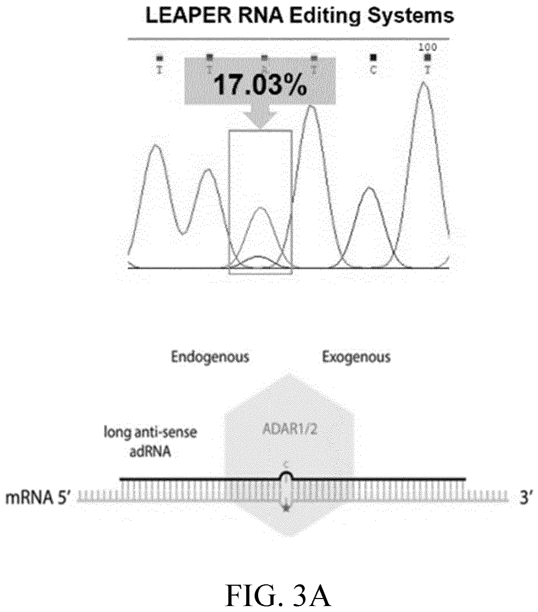

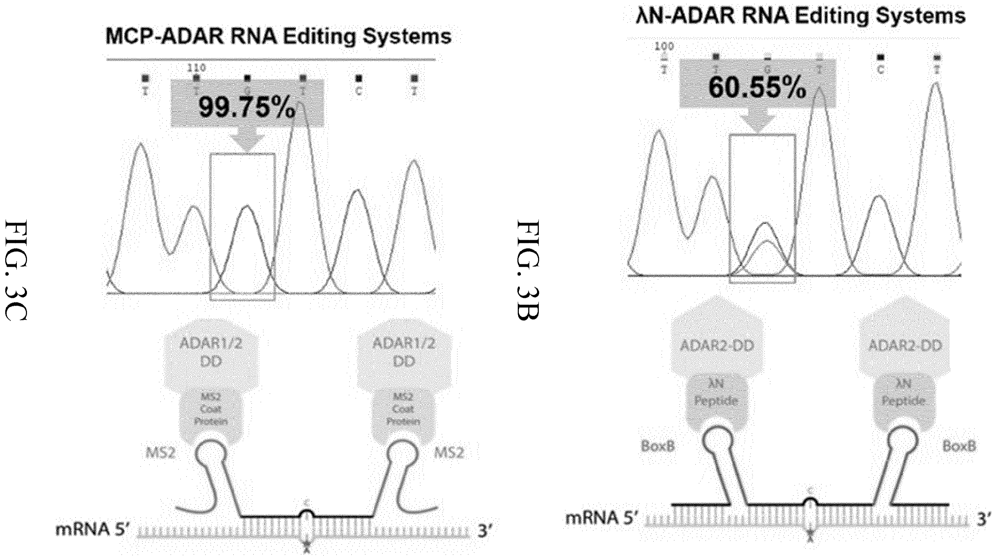

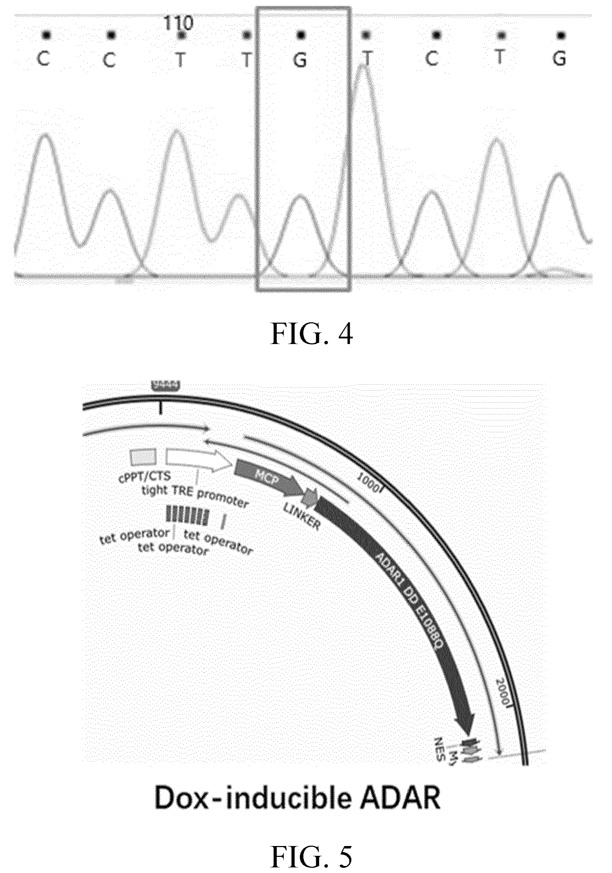

(4) Validation of in vitro editing efficiency: Hela cells were plated in a 12-well plate, and the guide RNA plasmids (plasmids from steps (1)-(3), respectively, plasmids based on the MS2-MCP2 system: 2500 ng of pUC57-arP1A-MS2 and 250 ng of pcDNA3.1(-)-U6-MS2-ADAR1-TAG; plasmids based on the BoxB system: 2500 ng of pUC57-arP1A-BoxB and 250 ng of pcDNA3.1(-)-U6-LN-ADAR2-TAG; plasmid based on the LEAPER system: 250 ng of pLKO-p1a-ar16) and 125 ng of P1A overexpression plasmid (pLKO-P1A-P2A-EGFP, sequence shown as SEQ ID NO: 27) (FIG. 2) were co-transfected using lipo8000 (Beyotime), and cells were lysed to extract RNA after 48 h. cDNA was obtained by RT-PCR, PCR was performed using primer(s) for P1A (P1A-1F, P1A-RTR2 in Table 3), the PCR products were subjected to Sanger sequencing to validate editing efficiency, and the validation results were as shown in FIGs. 3A-3C. The MS2-MCP2 system exhibited the highest editing efficiency, thus, subsequent experiments were performed using this system. Using the same method, the effectiveness of the MS2-MCP2 system in inducing RNA editing was validated in mouse CT26 cells, with the editing effect shown in FIG. 4. The results showed that the scheme of the present invention could effectively induce the occurrence of RNA editing.

**Table 2**

| Target | Design method | Guide RNA sequence |
|---|---|---|
| P1A | MS2 | AACATGAGGATCACCCATGTCctagaCaaggcagaatttcttAACATGAGG ATCACCCATGTC（SEQ ID NO：5） |
| P1A | LEAPER | tcataaagggcgtctatgaacatctggagcgccagaaaacttgttgtgacaacagcgaagaccagccac cctagaCaaggcagaatttcttccagggagtaccggtgcaataaattgcacctattcccatcaccgtcacc acctgagccac（SEQ ID NO：6） |
| P1A | BOXB | cagcgaagacggccctgaaaaagggccccaccctagaCaaggcagaatttcttccagggccctgaaa aagggccgtaccggtgc（SEQ ID NO：7） |

### 3. Establishment of a mouse model for intratumor induction of REISN.

Mouse model for intratumor induction of REISN was established, with a specific process as follows:
(1) Construction of Dox-inducible P1A RNA editing cell lines:
   1) ADAR lentiviral vector was constructed, with a diagram of the vector as shown in FIG. 5. The specific construction method was as follows: pCW57-MCS1-P2A-MCS2 (Addgene, #89180) plasmid was modified, and the ADAR1-MCP gene (sequence shown as SEQ ID NO: 24) was respectively inserted between restriction enzyme cutting sites of NheI and MluI downstream of the tight TRE promoter using Gibson cloning (ClonExpress^{®} II One Step Cloning Kit, Vazyme, C112) method, to obtain the Dox-inducible PCW-ADAR1-PURO (Dox-inducible ADAR) lentiviral vector.
   2) Guide RNA lentiviral expression vector was constructed, plasmid of pLKO.1 puro vector (Addgene, #8453) was modified, arP1A (sequence shown as SEQ ID NO: 5, which was synthesized by Tsingke Biotechnology Co., Ltd.) was cloned into between restriction enzyme cutting sites of AgeI and EcoRI downstream of the U6 promoter, and the puro antibiotic tag was replaced with BSD (product: blasticidin S deaminase), to obtain the guide RNA expression vector pLK0.1-arP1A-MS2-BLAST.
   3) Since CT26 cells do not naturally express P1A, an overexpression lentiviral vector pLKO-P1A-P2A-EGFP was designed (the sequence of P1A-P2A-EGFP (sequence shown as SEQ ID NO: 28) was cloned into between NheI and SalI of the pLKO.1 puro vector (Addgene, #8453)) (diagram as shown in FIG. 2). After co-transfection of 293T cells with packaging plasmids, CT26 cells were infected with the supernatant containing lentivirus, and then positive cells (i.e. CT26-P1A cells) were sorted out via fluorescent channel FITC.

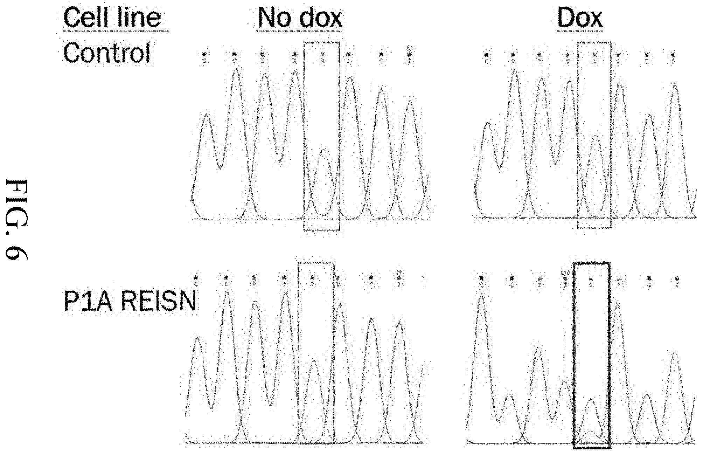

Lentiviral vectors obtained from steps 1) and 2) were co-transfected into 293T cells with packaging plasmids, and supernatant containing lentivirus was harvested. CT26-P1A cells were infected with 500 µL of the above Dox-inducible ADAR, arP1A lentiviral supernatant together with 10 µg/mL protamine. After 48 h, Puromycin with a final concentration of 12 µg/mL and Blastidin with a final concentration of 6 µg/mL were added for screening, and a stable cell line of CT26-P1A-REISN was obtained. After inducing the cell line with 0.5 µg/mL doxycycline, RNA was extracted and subjected to RT-PCR (PCR conditions: 95 °C, 3 min; 95 °C, 15 s; 60 °C, 15 s; 72 °C, 1 min; cycles 40; 72 °C, 5 min; 4 °C). The PCR primers were as shown in Table 3. Sanger sequencing was performed. The results were shown in FIG. 6. FIG. 6 showed the results of dox-free (left) and with the addition of dox (right), respectively, demonstrating that arP1A could successfully realize A-to-I RNA editing in vitro by dox induction.

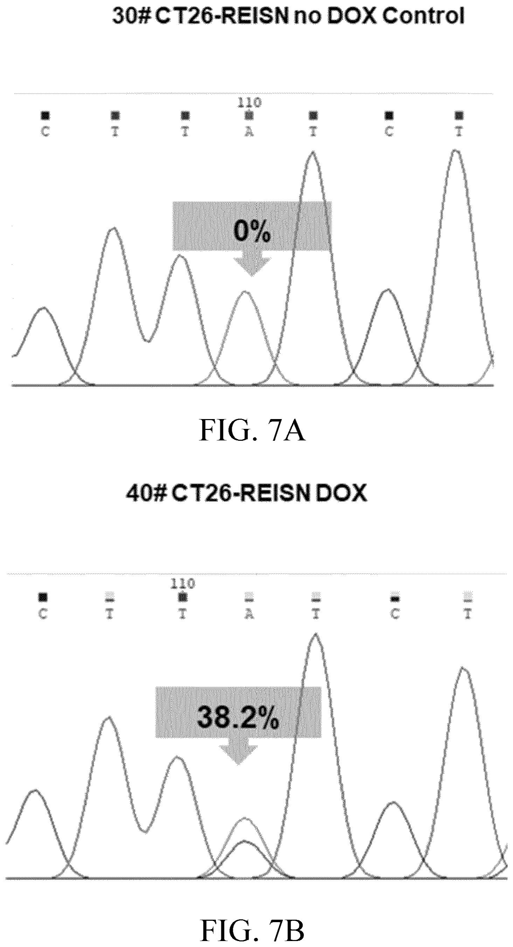

Balb/C mice (n = 3) were subcutaneously injected with 5×10⁵ CT26-P1A-REISN cells. After the tumor size reached a certain size (70-100 mm³), a diet containing doxycycline (0.2% doxycycline) was fed to induce REISN expression following RNA editing. After 10 days, tumor tissue was collected, total RNA was extracted (treated with DNase digestion) according to the instruction of the kit, reverse transcription to cDNA (further DNase digestion) was performed by RT-PCR, the target site was amplified by PCR using corresponding primers, and the PCR products were used for Sanger sequencing. FIGs. 7A and 7B showed the sequencing results of the tumor tissues from normal mouse diet and dox-added mouse diet, respectively, demonstrating that arP1A could successfully realize A-to-I RNA editing in vivo by dox induction. RNA editing efficiency for intratumor induction was about 38.2% (green peaks represent A before editing, black peaks represent G after editing, and peak heights were measured by QSV analysis software, editing efficiency %=G/(A+G)).

**Table 3**

| Primer name | Sequence |
|---|---|
| P1A-1F | Tgataacaagaagccagacaaagc (SEQ ID NO: 8) |
| P1A-RTR2 | ccaggaaattagggtcgtggaag (SEQ ID NO: 9) |

### (3) Immunotherapy with neoantigen vaccine targeting REISN epitope in tumor-bearing mice

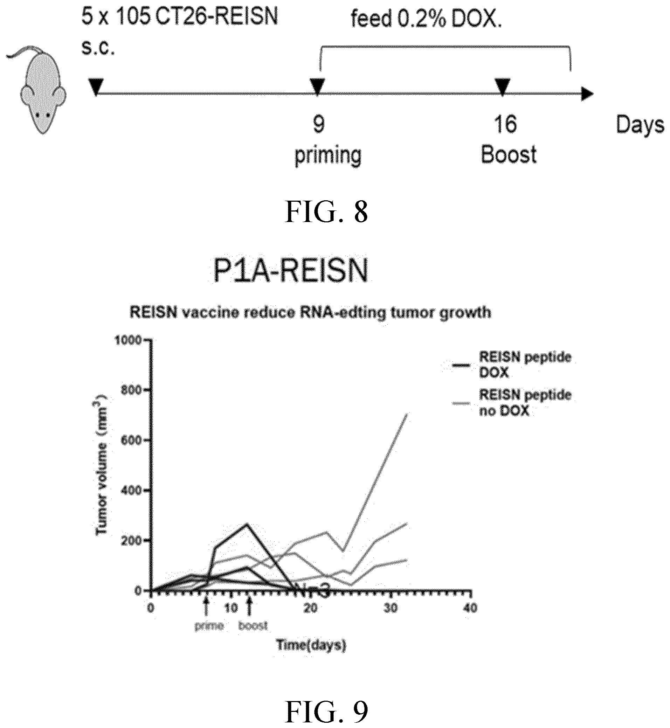

Balb/C mice (n = 3) were subcutaneously injected with 5×10⁵ CT26-pla-REISN cells. After the tumor size reached a certain size (70-100 mm³), the mice were immunized by pulsing spleen cells (post-irradiation) with 2×10⁷ short peptides of the neoantigen, and were fed with a diet containing doxycycline (0.2% doxycycline) to induce REISN expression following RNA editing. The same intensive immunization by pulsing spleen cells with 2×10⁷ short peptides of the neoantigen was performed one week later, and the detection procedure was as shown in FIG. 8. Tumor growth was assessed by measuring the size of the subcutaneous tumor.

The detection results were as shown in FIG. 9, from which it can be seen that the tumors of the tumor-bearing mice were regressed after REISN immunotherapy, indicating that immunotherapy with the neoantigen vaccine can target and kill the tumor cells expressing REISN.

### Example 3

In the present example, a preparation method of the antigenic epitope was validated using murine Spag9-2 gene as an example, with a specific process as follows:

### 1. Design of the REISN site

For the murine Spag9-2 gene, binding affinities of peptides of all the protein sequences in the ORF of the gene for the mouse MHC H-2-Kd were predicted by using the NetMHCpan-4.1 software. A Spag9-2 short peptide with high affinity of 484.06 nM for H-2-Kd was identified, and among the adenine nucleotides in this peptide segment, a mutant peptide Spag9-2 -REISN that could change the amino acid coding after mutating to guanine was identified. The NetMHCpan-4.1 software was used to predict the binding affinity of the mutant peptide for H-2-Kd. Sequence and the prediction result of the affinity were as shown in Table 4 (the method of the present example predicted MHC-class I complexes).

**Table 4**

| Protein name | Peptide | MHC | Aff(nM) |
|---|---|---|---|
| Spag9-2 | VYKDQISVL (SEQ ID NO: 10) | H-2-Kd | 484.06 |
| Spag9-2 -REISN | VYKDQMSVL (SEQ ID NO: 11) | H-2-Kd | 651.29 |

### 2. Design of REISN guide RNA

(1) Guide RNA design based on MS2-MCP2 system: MS2-MCP2 system consisted of two components: exogenous ADAR1 and guide RNA (sequence shown as SEQ ID NO: 12 in Table 5). The construction method was consistent with that in Example 2.

**Table 5**

| targe t | Design method | Guide RNA sequence |
|---|---|---|
| Spag 9-2 | MS2 | AACATGAGGATCACCCATGTCACTGAcATTTGATCTTTGTACAACAT GAGGATCACCCATGTC（SEQ ID NO：12） |

### 3. Establishment of the mouse model for intratumor induction of REISN.

Mouse model for intratumor induction of REISN was established, with a specific process as follows:
(1) Construction of Dox-inducible Spag9-2 RNA editing cell lines:
   1) ADAR lentiviral vector was constructed, with a diagram of the vector as shown in FIG. 5. The construction method was consistent with that in Example 2.
   2) Guide RNA lentiviral expression vector of pLKO.1-arSPAG9-2-MS2-BLAST was constructed. The construction method was consistent with that in Example 2.

Lentiviral vectors obtained from steps 1) and 2) were co-transfected into 293T cells with packaging plasmids, and supernatant containing lentivirus was harvested. CT26 cells were infected with 500 µL of the above Dox-inducible ADAR, arSPAG9-2 lentiviral supernatant together with 10 µg/mL protamine. After 48 h, Puromycin with a final concentration of 12 µg/mL and Blastidin with a final concentration of 6 µg/mL were added for screening, and a stable cell line of CT26-SPAG9-2-REISN was obtained.

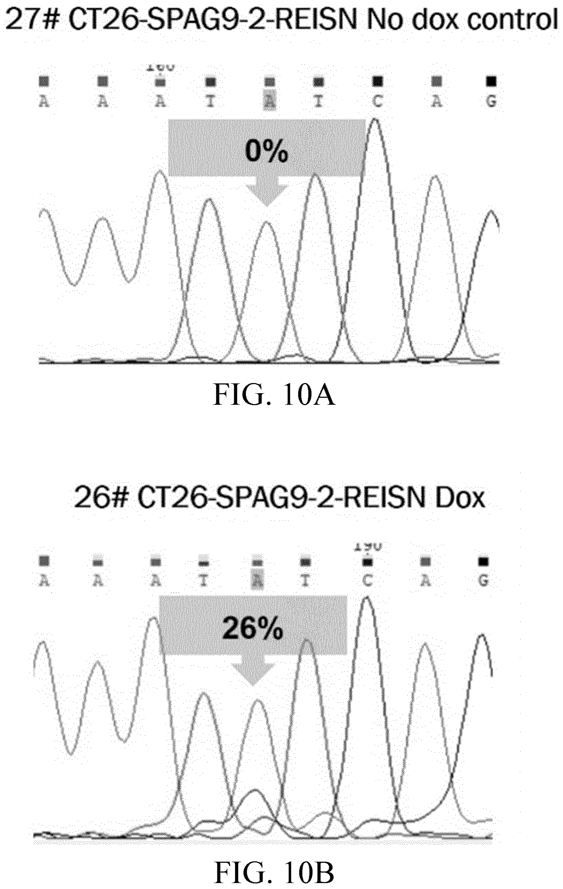

Balb/C mice (n = 3) were subcutaneously injected with 5×10⁵ CT26-SPAG9-2-REISN cells. After the tumor size reached a certain size (70-100 mm³), a diet containing doxycycline (0.2% doxycycline) was fed to induce REISN expression following RNA editing. Tumor tissue was collected, total RNA was extracted (treated with DNase digestion) according to the instruction of the kit, reverse transcription to cDNA (further DNase digestion) was performed by RT-PCR, the target site was amplified by PCR using corresponding primers (sequences as shown in Table 6), and the PCR products were used for Sanger sequencing. FIGs. 10A-10B showed the sequencing results, from which it can be seen that arSpag9-2could successfully realize A-to-I RNA editing in vivo by dox induction. RNA editing efficiency for intratumor induction was about 26%.

**Table 6**

| Primer name | Sequence |
|---|---|
| arSpag9-2/3-1F | GCAGAAGAAGCAACTGAAGCC (SEQ ID NO: 13) |
| arSpag9-2/3-1R | TGCAAGACACCATAAGAGCTG (SEQ ID NO: 14) |

### (2) Immunotherapy with neoantigen vaccine targeting REISN epitope in tumor-bearing mice

Balb/C mice (n = 5) were subcutaneously injected with 5×10⁵ CT26-SPAG9-2-REISN cells. After the tumor size reached a certain size (70-100 mm³), the mice were immunized by pulsing spleen cells (post-irradiation) with 1×10⁷ short peptides of the neoantigen, and were fed with a diet containing doxycycline (0.2% doxycycline) to induce REISN expression following RNA editing. Tumor growth was assessed by measuring the size of the subcutaneous tumor every 3 days.

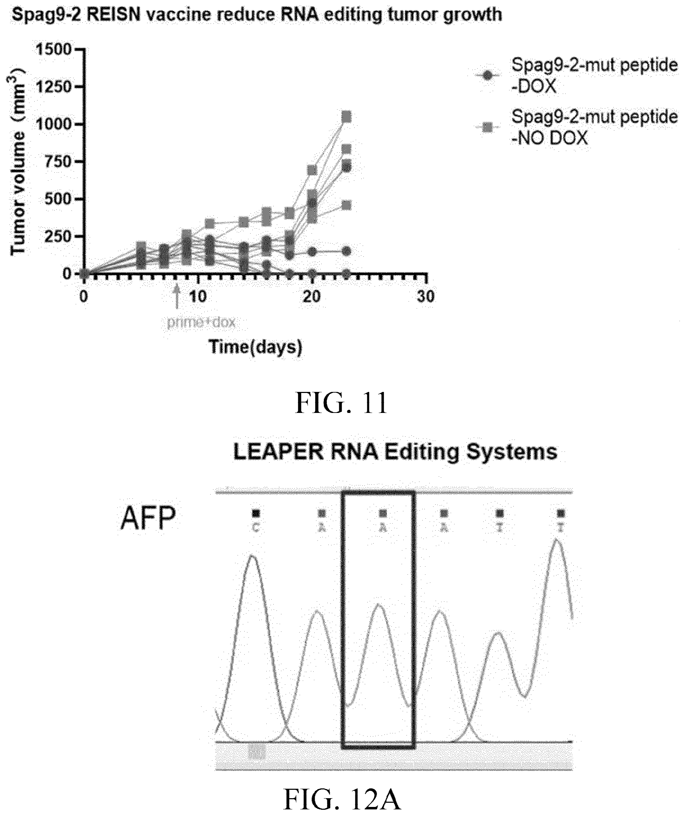

The detection results were as shown in FIG. 11, from which it can be seen that the tumors of the tumor-bearing mice were regressed after REISN immunotherapy, indicating that immunotherapy with the neoantigen vaccine can target and kill the tumor cells expressing REISN.

### Example 4

In the present example, a preparation method of the antigenic epitope was validated using human AFP gene as an example, with a specific process as follows:

### 1. Design of the REISN site

For the human AFP gene, binding affinities of peptides of all the protein sequences in the ORF of the gene for human HLA-A*02:01 were predicted by using the NetMHCpan-4.1 software. An AFP short peptide with high affinity for HLA-A*02:01 was identified, and among the adenine nucleotides in this peptide segment, three mutant peptides that could change the amino acid coding after mutating to guanine was identified (sequences as shown in Table 7). The NetMHCpan-4.1 software was used to predict the binding affinity of the mutant peptides for HLA-A*02:01. Sequences and the prediction results of the affinity were as shown in Table 7 (the method of the present example predicted MHC-class I complexes).

**Table 7**

| Protein name | Peptide | MHC | Aff(nM) |
|---|---|---|---|
| AFP | FMNKFIYEI (SEQ ID NO: 15) | HLA-A*02:01 | 2.2 |
| AFP -REISN1 | FMNKFVYEI (SEQ ID NO: 16) | HLA-A*02:01 | 2.3 |
| AFP -REISN2 | FMNKFICEI (SEQ ID NO: 17) | HLA-A*02:01 | 4.7 |
| AFP -REISN3 | FMNRFIYEI (SEQ ID NO: 18) | HLA-A*02:01 | 2.2 |

### 2. Design of REISN guide RNA

(1) Guide RNA design based on MS2-MCP2 system: MS2-MCP2 system consisted of two components: exogenous ADAR1 and guide RNA (sequence shown as SEQ ID NO: 20). Two plasmids, pcDNA3.1(-)-MCP-ADAR1-TAG and pUC57-arAFP-MS2, were constructed, both of which were synthesized by Nanjing GenScript Biotechnology Co., Ltd, and wherein the cloning process of pcDNA3.1(-)-MCP-ADAR1-TAG was as shown in Example 2.
   pUC57-arAFP-MS2 was constructed by inserting U6 promoter and arAFP-MS2 fragment (sequence shown as SEQ ID NO: 20) between restriction enzyme cutting sites of EcoRI and HindIII in the MCS region of the pUC57 vector. Sequencing verified that the sequence was correct and complete.
(2) Guide RNA design based on LEAPER system: LEAPER editing system consisted of guide RNA (i.e. ar13, sequence shown as SEQ ID NO: 19). pLKO-pla-ar13 plasmid was constructed. This plasmid was constructed by cloning U6 promoter and the synthesized ar13 fragment (sequence shown as SEQ ID NO: 19) into between XbaI and SalI of pLKO.1 puro (Addgene #8453) backbone via homologous recombination.
(3) Guide RNA design based on λN-BoxB system: λN-BoxB editing system consisted of exogenous ADAR2 and guide RNA (i.e. arAFP, sequence shown as SEQ ID NO: 21). pUC57-arAFP-BOXB and pcDNA3.1(-)-U6-BOXB-ADAR2 plasmids were constructed, both of which were synthesized by Nanjing GenScript Biotechnology Co., Ltd, and wherein the cloning process of pcDNA3.1(-)-U6-BOXB-ADAR2 was as shown in Example 2. The pUC57-arAFP-BoxB was obtained by inserting the U6 promoter and arAFP-BoxB fragment (sequence shown as SEQ ID NO: 21) between restriction enzyme cutting sites of EcoRI and HindIII in the MCS region of the pUC57 backbone vector. Sequencing verified that the sequence was correct and complete.
(4) Validation of in vitro editing efficiency: since Hela cells do not express AFP, the AFP overexpression plasmid pLKO-AFP-EGFP was constructed. The pLKO-AFP-EGFP plasmid was constructed by cloning AFP-EGFP sequence (sequence shown as SEQ ID NO: 28) between NheI and SalI of pLKO.1 puro backbone vector (Addgene, #8453). For the LEAPER editing system, Hela cells were plated in a 96-well plate, 250 ng of pLKO-AFP-ar13 plasmid and 12.5 ng of pLKO-AFP-EGFP plasmid were co-transfected using lipo8000 (Beyotime), and cells were lysed to extract RNA after 48 h. cDNA was obtained by RT-PCR, PCR was performed using primer(s) for AFP (AFPRT F1: CAGCCAAAGTGAAGAGGGAAGA (SEQ ID NO: 22), AFPRT R1: TTTTCCCCATCCTGCAGACAAT (SEQ ID NO: 23)), the PCR products were subjected to Sanger sequencing to validate editing efficiency; for the MS2 and BoxB systems, Hela cells were plated in a 12-well plate, 2.5 µg of guide RNA plasmid, 250 ng of ADAR plasmid, and 125 ng of AFP overexpression plasmid (pLKO-AFP-EGFP) were simultaneously transfected into cells with Lipo800 transfection reagent. Cells were lysed to extract RNA after 48 h, cDNA was obtained by RT-PCR, and PCR was performed using primer(s) for AFP (AFPRT F1, AFPRT R1), the PCR products were subjected to Sanger sequencing to validate editing efficiency.

**Table 8**

| Target | Design method | Guide RNA sequence |
|---|---|---|
| AFP | LEAPER | ATAGCGAGCAGCCCAAAGAAGAATTGTAGGTGCATACAGGAAGG GATGCCTTCTTGCTATCTCATAAATGAATCTGTTCATGAATGTCTC CCTGTCTTCTTCATATGCTTCACAGCTTGTGACAGGTTCTGGAACT TGGAAAAGTGGGATC（SEQ ID NO：19） |
| AFP | MS2 | AACATGAGGATCACCCATGTCTGAATCTGTTCATGAATGTCTAAC ATGAGGATCACCCATGTC（SEQ ID NO：20） |
| AFP | BoxB | TTCTTGCTATGgccctgaaaaagggccATAAATGAATCTGTTCATGAATGT CTCCCTggccctgaaaaagggccttCTTCATAT（SEQ ID NO：21） |

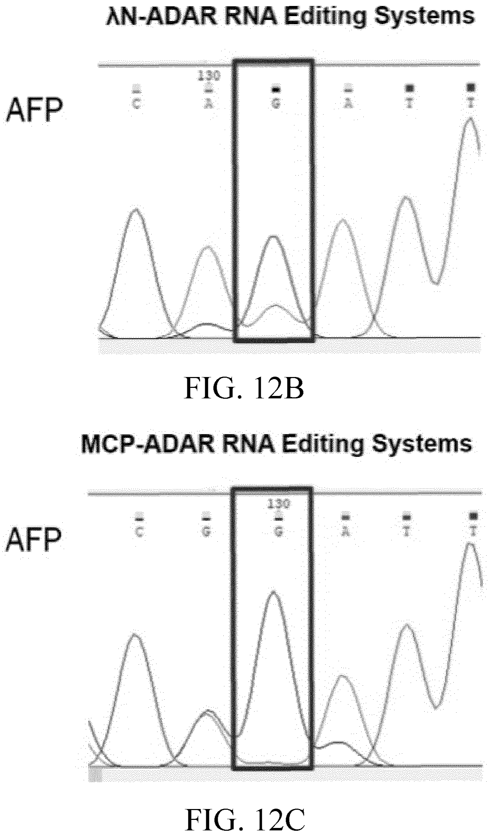

As can be seen from FIGs. 12A-12C, both of the MS2 and BoxB systems could effectively perform gene editing.

The gene fragments and plasmids in the embodiments of the present invention were all synthesized by Nanjing GenScript Biotechnology Co., Ltd.

The examples of the present invention have been described in detail as above in conjunction with the accompanying drawings, however, the present invention is not limited to the examples as above. Various changes can be made within the scope of knowledge possessed by a person of ordinary skill in the art, without departing from the purpose of the present invention. Furthermore, the examples of the present invention and the features in the examples may be combined with each other without conflict.

## Claims

1. A preparation method for an antigenic epitope, wherein the preparation method comprises the following step: using RNA editing technology to perform site-directed mutation on a tumor antigen to obtain a neoantigenic epitope.

2. The preparation method according to claim 1, wherein the site-directed mutation is achieved by mutating A to I and/or C to U in a nucleic acid sequence of RNA of the tumor antigen.

3. The preparation method according to claim 1, wherein the tumor antigen comprises a tumor-specific antigen or a tumor-associated antigen.

4. The preparation method according to claim 3, wherein the tumor-associated antigen comprises at least one of an embryonic antigen, a differentiation antigen, a cancer testis antigen, or a viral antigen.

5. The preparation method according to claim 3, wherein the tumor-specific antigen comprises at least one of a chemically-induced tumor-specific antigen, a viral-induced tumor-specific antigen, or an antigen of a spontaneous tumor.

6. The preparation method according to claim 1, wherein an affinity of the neoantigenic epitope for binding to MHC is not less than 1000 nM.

7. The preparation method according to claim 1, wherein an editing system employed by the RNA editing technology comprises a guide RNA construct; preferably, guide RNA expressed by the construct has a sequence of not less than 16 bp, is reverse complementary to a sequence of a mRNA target site, and has a homology of not less than 85%.

8. The preparation method according to claim 1, wherein the editing system employed by the RNA editing comprises at least LEAPER, λN-BoxB, or MS2-MCP2.

9. An antigenic epitope, wherein the antigenic epitope is prepared by the preparation method according to any one of claims 1-8.

10. The antigenic epitope according to claim 9, wherein an amino acid sequence of the antigenic epitope is shown as SEQ ID NO: 3, SEQ ID NO: 11, SEQ ID NO: 16, SEQ ID NO: 17 or SEQ ID NO: 18.
